# EUROPEAN PATENT SPECIFICATION

(11) **EP 4 264 232 B1**
(45) Date of publication and mention of the grant of the patent: **15.10.2025**
(21) Application number: 21843675.6
(22) Date of filing: 20.12.2021
(51) Int. Cl.: G01N 21/03, G01N 21/3577, G01N 21/39, A61B 5/08, A61B 5/145, A61B 5/20, A61B 5/00, A61B 5/083

(54) **TOTAL ENERGY EXPENDITURE MEASUREMENTS WITH H2 18O DEPLETED WATER**
MESSUNG DES GESAMTENERGIEVERBRAUCHS MIT H2 18O ABGEREICHERTEM WASSER
MESURES DE LA DÉPENSE ÉNERGÉTIQUE TOTALE AVEC DE L'EAU APPAUVRIE EN H2 18O

(30) Priority: 21.12.2020 EP 20216104
(43) Date of publication of application: 25.10.2023
(73) Proprietor: JOANNEUM RESEARCH Forschungsgesellschaft mbH, 8010 Graz (AT); JR-AquaConSol GmbH, 8010 Graz (AT)
(72) Inventor: LEIS, Albrecht, 8045 Graz (AT); MAGNES, Christoph, 8072 Fernitz-Mellach (AT); EBERL, Anita, 8302 Nestelbach (AT); SCHWINGENSCHUH, Simon, 8010 Graz (AT)
(74) Representative: Geling, Andrea
(86) International application number: PCT/EP2021/086840
(87) International publication number: WO 2022/136293

(56) References cited:
- KLEIN S ET AL: "Energy metabolism inresponse to overfeeding in young adult men", METABOLISM, CLINICAL AND EXPERIMENTAL, W.B. SAUNDERS CO., PHILADELPHIA, PA, US, vol. 42, no. 9, 1 September 1993 (1993-09-01), pages 1201 - 1205, XP023312141, ISSN: 0026-0495, [retrieved on 19930901], DOI: 10.1016/0026-0495(93)90281-R
- SCHOELLER D A ET AL: "ENERGY EXPENDITURE BY DOUBLY LABELED WATER: VALIDATION IN HUMANS AND PROPOSED CALCULATION", AMERICAN JOURNAL PHYSIOL RENAL PHYSIOL, AMERICAN PHYSIOLOGICAL SOCIETY, US, vol. 250, no. 5, PART 02, 1 January 1986 (1986-01-01), pages R823 - R830, XP009068937, ISSN: 0002-9513
- GORAN M I ET AL: "Variation in total energy expenditure in young healthy free-living men", METABOLISM, CLINICAL AND EXPERIMENTAL, W.B. SAUNDERS CO., PHILADELPHIA, PA, US, vol. 42, no. 4, 1 April 1993 (1993-04-01), pages 487 - 496, XP026359568, ISSN: 0026-0495, [retrieved on 19930401]
- KOEA ET AL: "Total energy expenditure during total parenteral nutrition: Ambulatory patients at home versus patients with sepsis in surgical intensive care", SURGERY, MOSBY, INC, US, vol. 118, no. 1, 1 July 1995 (1995-07-01), pages 54 - 62, XP005437069, ISSN: 0039-6060, DOI: 10.1016/S0039-6060(05)80010-8
- GORAN M I ET AL: "Total energy expenditure and energy requirements in healthy elderly persons", METABOLISM, CLINICAL AND EXPERIMENTAL, W.B. SAUNDERS CO., PHILADELPHIA, PA, US, vol. 41, no. 7, 1 July 1992 (1992-07-01), pages 744 - 753, XP023312292, ISSN: 0026-0495, [retrieved on 19920701], DOI: 10.1016/0026-0495(92)90315-2

## Description

The present invention relates to a combination comprising ²H₂O enriched water and H₂¹⁸O depleted water. Further, the present invention relates to a kit for measuring energy expenditure of a subject, said kit comprises the combination comprising ²H₂O enriched water and H₂¹⁸O depleted water. Furthermore, the present invention relates to the use of said combination or kit for measuring energy expenditure of a subject. In addition, the present invention relates to a method of measuring energy expenditure of a subject.

### BACKGROUND OF THE INVENTION

Various calorimetric methods are used in medicine to determine the total energy turnover of a person. The measurement of the energy turnover by means of so-called direct calorimetry is extremely complex. In clinical studies, indirect calorimetry is often performed selectively using breathing masks with carbon dioxide (CO₂) and oxygen (O₂) sensors. This method is very accurate, but does not provide measured values over a longer period of time, as the test persons cannot be expected to wear the masks for longer.

Therefore, indirect methods are usually used for determining energy expenditure, in which the energy expenditure is calculated from the oxygen (O₂) uptake and/or the carbon dioxide (CO₂) emission.

Carbon dioxide (CO₂) release can also be determined by enriching the stable isotopes ¹⁸O and deuterium (²H) in body water and determining the elimination rates of both isotopes from the body by isotope ratio determination. The underlying principle is that deuterium (²H) is depleted only by water exchange with the environment, while oxygen (O₂) is also equilibrated with the environment via carbon dioxide (CO₂). This method - the so-called Doubly Labeled Water (DLW) method - has been known since the 1950s and has also been applied to humans since the 1980s. It is currently used in clinical studies. The method enables the determination of energy expenditure over a period of up to two weeks with little effort on the part of the test persons. The test persons must provide a total of 3 urine samples distributed over the measurement period and drink isotopically enriched water at the beginning. The application is currently still limited by the high costs of the ¹⁸O isotope tracer.

In the sport, training and lifestyle sector there are currently many applications that estimate calorie consumption via pedometer, pulse sensor etc. However, these methods are extremely imprecise.

Thus, there is an unmet need for a new (Doubly Labeled Water (DLW)) method for determining energy expenditure/consumption which is cost-effective as well as easy and broadly applicable.

The present inventors surprisingly found that H₂¹⁸O depleted water can be used in a method for determining (total) energy expenditure. Due to the administration of H₂¹⁸O depleted water to a subject, the natural concentration of H₂¹⁸O in body water is slightly reduced. The H₂¹⁸O depleted body water further equilibrates via the water ingested during food intake and CO₂ exchange during respiration until it reaches the original natural H₂¹⁸O concentration again. From this, the elimination rate of ¹⁸O and, thus, the CO₂ turnover can be determined. The CO₂ turnover then allows to determine the (total) energy expenditure.

H₂¹⁸O depleted water is significantly cheaper to produce than H₂¹⁸O enriched water. It is a waste product of ¹⁸O isotope enrichment. In this way, a cost reduction of the method by an estimated factor of 10 can be achieved. This also makes the method interesting for private consumption (sport, training, nutrition, and/or lifestyle).

The method performed by the present inventors allows the determination of energy expenditure which high accuracy and precision. It can also be used to calibrate the inaccurate methods described in the prior art for each person individually.

In addition, the present inventors surprisingly found that ¹⁷O can be used as a tracer for background correction. Due to the additional use of H₂¹⁷O depleted or enriched water, the detection sensitivity can be improved.

### SUMMARY OF THE INVENTION

In a first aspect, the present invention relates to a combination comprising ²H₂O enriched water and H₂¹⁸O depleted water.

In a second aspect, the present invention relates to a kit for measuring energy expenditure of a subject, said kit comprises: the combination of the first aspect.

In a third aspect, the present invention relates to the use of the combination of the first aspect or the kit of the second aspect for measuring energy expenditure of a subject.

In a fourth aspect, the present invention relates to a method of measuring energy expenditure of a subject comprising the steps of:
(i) measuring the ²H/¹H ratio and the ¹⁸O/¹⁶O ratio in a body water sample obtained from a subject at a first point in time (pre-dose baseline ratios),
(ii) measuring the ²H/¹H ratio and the ¹⁸O/¹⁶O ratio in a body water sample obtained from the subject at a second point in time (post-dose ratios) and in at least one further body water sample obtained from said subject at a later point in time, wherein the subject is a subject to whom a combination comprising ²H₂O enriched water and H₂¹⁸O depleted water had been administered,
(iii) determining a combined value of flux of body water and exhaled/released carbon dioxide (CO₂) from a change in measured ¹⁸O/¹⁶O ratios over time and a value of flux of body water alone from a change in measured ²H/¹H ratios over time, and
(iv) calculating the energy expenditure on the basis of the exhaled/released CO₂ over time calculated from the combined values determined in step (iii).

In a fifth aspect, the present invention relates to a method of training optimization comprising the steps of:
(i) carrying out the method of the fourth aspect, thereby measuring the energy expenditure of a subject, and
(ii) adapting the training of the subject on the basis of the energy expenditure measured in step (i).

In a sixth aspect, the present invention relates to a method of diet optimization comprising the steps of:
(i) carrying out the method of the fourth aspect, thereby measuring the energy expenditure of a subject, and
(ii) adapting the diet of the subject on the basis of the energy expenditure measured in step (i).

In a seventh aspect, the present invention relates to a method of obesity therapy optimization comprising the steps of:
(i) carrying out the method of the fourth aspect, thereby measuring the energy expenditure of a subject, and
(ii) adapting the obesity therapy of the subject on the basis of the energy expenditure measured in step (i).

This summary of the invention does not necessarily describe all features of the present invention. Other embodiments will become apparent from a review of the ensuing detailed description.

### DETAILED DESCRIPTION OF THE INVENTION

### Definitions

It is to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art.

Preferably, the terms used herein are defined as described in "A multilingual glossary of biotechnological terms: (IUPAC Recommendations)", Leuenberger, H.G.W, Nagel, B. and Kölbl, H. eds. (1995), Helvetica Chimica Acta, CH-4010 Basel, Switzerland).

Several documents are cited throughout the text of this specification.

The term "comprise" or variations such as "comprises" or "comprising" according to the present invention means the inclusion of a stated integer or group of integers but not the exclusion of any other integer or group of integers. The term "consisting essentially of" according to the present invention means the inclusion of a stated integer or group of integers, while excluding modifications or other integers which would materially affect or alter the stated integer. The term "consisting of" or variations such as "consists of" according to the present invention means the inclusion of a stated integer or group of integers and the exclusion of any other integer or group of integers.

The terms "a" and "an" and "the" and similar reference used in the context of describing the invention (especially in the context of the claims) are to be construed to cover both the singular and the plural, unless otherwise indicated herein or clearly contradicted by context.

The term "(total) energy expenditure (TEE)", as used herein, refers to the amount of energy a subject uses to maintain essential body functions (respiration, circulation, digestion) and to be physically active. (Total) energy expenditure can be partitioned into three components, the basal metabolism, the diet-induced thermogenesis, and the muscular contraction. However, the relative contribution of each component to (total) energy expenditure is largely dependent upon the interindividual variability of each component. For example, (total) energy expenditure from physical activity is the most variable.

The term "basal metabolism", as used herein, refers to the first and primary component of (total) energy expenditure. It comprises 60% to 70% of (total) energy expenditure. The basal metabolism is defined as the turnover of energy in a fasting and resting organism using energy solely to maintain vital cellular activity, respiration, and circulation as measured by the basal metabolic rate (BMR).

The term "diet-induced thermogenesis" as used herein, refers to the second component of (total) energy expenditure. The diet-induced thermogenesis is defined as the energy required to digest, absorb, and store food. Adaptive thermogenesis or the energy required to thermoregulate and respond to changes in the environmental temperature comprises 10% to 15% of (total) energy expenditure.

The term "muscular contraction", as used herein, refers to the third and most variable component of (total) energy expenditure. It comprises 6% to 10% of (total) energy expenditure. The muscular contraction is defined as physical activity thermogenesis or the work derived from all forms of physical activity. This includes both physical activity due to exercise and all other types of physical activity excluding exercise (e.g. spontaneous physical activity or non-exercise activity).

The components of (total) energy expenditure and measurement approaches are summarized in

### Figure 1.

Please note that the terms "energy expenditure" and "energy consumption" are interchangeable used herein.

(Total) energy expenditure can be estimated by measuring macronutrient or oxygen consumption, or heat production or carbon dioxide production. Since measuring the heat generated during physical activity (direct calorimetry) is extremely complex, indirect methods are usually used in practice, in which energy expenditure is calculated from oxygen uptake and/or carbon dioxide release. One such method is the Doubly Labeled Water (DLW) method.

The term "Doubly Labeled Water (DLW) method", as used herein, refers to a method allowing the determination of (total) energy expenditure of a subject. It is widely acknowledged as the criterion or "gold standard" approach to assess (total) energy expenditure. The technique is applicable in a wide range of populations including the most vulnerable such as pregnant and lactating women and infants. Importantly, the technique is suitable for use in a free-living context, is non-invasive and imposes minimal participant burden. (Total) energy expenditure is typically assessed over a 7- to 14-day period (depending on the analysis approach and age of participant). In this method, a certain amount of water, which is isotopically labelled with stable hydrogen (²H) and oxygen (¹⁸O) isotopes, is added to the organism. After adding the isotopically labeled water, the absorbed water mixes with the body water after a short time. As a result, the isotope ratios of the water in the organism change in a characteristic way. In the course of time, both isotopes (²H as well as ¹⁸O) are gradually eliminated from the body. While the stable hydrogen isotope (²H) leaves the body only in the form of water and water vapor, the oxygen isotope (¹⁸O) is also excreted in the form of carbon dioxide (CO₂) during respiration. This results in a significantly higher elimination rate for the latter oxygen isotope. By periodically measuring the isotope ratios of hydrogen and oxygen in a body fluid/body water such as plasma, urine, saliva during a defined period of time, the relative difference between them can be used to determine the CO₂ emission/production. The rate of carbon dioxide emission/production can then be converted to (total) energy expenditure such as average (total) energy expenditure.

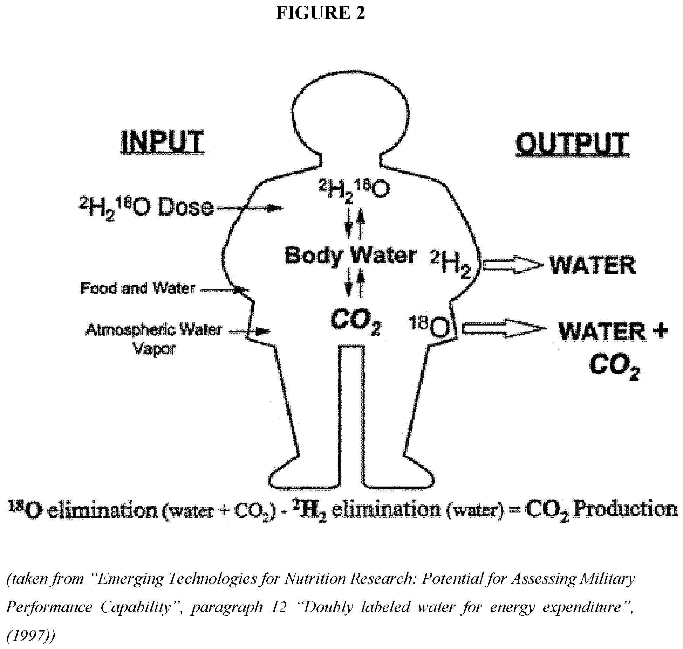

The readers are encouraged to access a number of recent International Atomic Energy Agency (IAEA) publications for a detailed overview of the DLW technique for the assessment of TEE and other isotopic techniques in nutrition and also recent papers in the area, see, for example Speakman J. R. "The history and theory of the doubly labeled water technique", Am J Clin Nutr (1998) 68(4): 932S-8S. An overview of the DLW method can be taken from **Figure 2****.**

Physically, stable isotopes are atoms which take the same position in the table of elements, but have a different number of neutrons and, therefore, mass. The most relevant isotopes for the determination of (total) energy expenditure are ¹⁸O for oxygen (corresponding to the most abundant isotope ¹⁶O), and ²H for hydrogen (corresponding to the most abundant isotope ¹H). The isotope ¹⁷O for oxygen is further known. The isotope ¹⁷O is less abundant in normal water than ¹⁸O.

The term "²H₂O enriched water", as used herein, refers to a form of water that contains more ²H (also designated as deuterium or D, also known as heavy hydrogen) than usually present therein. Generally, common ¹H isotope (also designated as hydrogen-1 isotope or H, also known as protium) makes up most of the hydrogen in normal water. The presence of the heavier hydrogen isotope gives the water an increased molecular mass with nearly the same physical and chemical properties when compared to normal water. In a preferred embodiment, ²H₂O enriched water contains between 0.02 % and 100% ²H₂O (specified as ²H fraction). For comparison, in the isotopic reference standard VSMOW (Vienna Standard Mean Ocean Water) only about 0.0156% of the hydrogen atoms are of the heavy type.

The term "deuterium", as used herein, refers to a hydrogen isotope with a nucleus containing a neutron and a proton; the nucleus of a protium (normal hydrogen) atom consists of just a proton. The additional neutron makes a deuterium atom roughly twice as heavy as a protium atom. A molecule of heavy water has two deuterium atoms in place of the two protium atoms of ordinary "light" water. The weight of a heavy water molecule, however, is not substantially different from that of a normal water molecule, because about 89% of the molecular weight of water comes from the single oxygen atom rather than the two hydrogen atoms.

The term "H₂¹⁸O depleted water", as used herein, refers to a form of water that contains less ¹⁸O than usually present therein. Generally, common ¹⁶O isotope makes up most of the oxygen in normal water. In a preferred embodiment, H₂¹⁸O depleted water contains between 0 % and < 0.2 % H₂¹⁸O (specified as ¹⁸O fraction). For comparison, in the isotopic reference standard VSMOW ≥ 0.2% of the oxygen atoms are of the ¹⁸O type.

The term "H₂¹⁷O enriched water", as used herein, refers to a form of water that contains more ¹⁷O than usually present therein. H₂¹⁷O enriched water contains between 0.037 % and 100 % H₂¹⁷O (specified as ¹⁷O fraction). Generally, common ¹⁶O isotope makes up most of the oxygen in normal water.

The term "H₂¹⁷O depleted water", as used herein, refers to a form of water that contains less ¹⁷O than usually present therein. Generally, common ¹⁶O isotope makes up most of the oxygen in normal water. H₂¹⁷O depleted water contains between 0 % and < 0.037 % H₂¹⁷O (specified as ¹⁷O fraction). For comparison, in the isotopic reference standard VSMOW about 0.0379 % of the oxygen atoms are of the ¹⁷O type.

The isotope ¹⁷O is preferably used herein as a new, additional tracer for the determination of (total) energy expenditure. There is a strong autocorrelation between all isotope ratios in natural water (²H/¹H, ¹⁷O/¹⁶O, ¹⁸O/¹⁶O). This autocorrelation can be used to correct for natural isotope ratio fluctuations. Ideally, the tracer used for the DLW-method should have nearly the natural abundance of ¹⁷O. Therefore, the measured ¹⁷O/¹⁶O ratio can be used to correct natural ²H/¹H and ¹⁸O/¹⁶O ratio fluctuation to improve the accuracy of the DLW method. The ¹⁷O/¹⁶O ratio can be determined from the parallel measurement of all three oxygen isotopes (¹⁶O, ¹⁷O, and ¹⁸O). This was made possible by a new generation of laser spectrometers, which have a significantly improved spectral resolution and sensitivity.

The term "¹⁸O/¹⁶O ratio", as used herein, refers to the relation of the isotopes ¹⁸O and ¹⁶O which are present/comprised in a body water sample obtained from a subject.

The term "²H/¹H ratio", as used herein, refers to the relation of the isotopes ²H and ¹H which are present/comprised in a body water sample obtained from a subject.

The term "¹⁷O/¹⁶O ratio", as used herein, refers to the relation of the isotopes ¹⁷O and ¹⁶O which are present/comprised in a body water sample obtained from a subject.

The term "¹⁷O-excess", as used herein, refers to the deviation of the ¹⁷O/¹⁶O ratio of a body fluid sample compared to the reference relationship between δ¹⁸O and δ¹⁷O of the "Global Meteoric Water Line".

The term "subject", as used herein, refers to any individual whose (total) energy expenditure is to be determined/measured. In particular, the subject is a hobby or professional athlete, an obese person, or a person suffering from a metabolic disorder such as diabetes or cardiovascular disease.

In one preferred embodiment, the subject is a human being or an animal. The animal may be selected from the group consisting of a dog, cat, sheep, goat, cow, horse, and pig. In one more preferred embodiment, the subject is a human being.

The terms "subject", "individual", "person", or "patient" are used interchangeably herein.

The term "body water sample", as used herein, refers to any watery/aqueous sample taken from a subject, e.g. human being. In other words, the body water (also designated as body fluid or bodily fluid) is any liquid within the body of a subject, e.g. human being. A body water sample can be obtained, for example, by collection of any of urine, blood, saliva and exhaled breath from a subject. Preferably, the body water sample is selected from the group consisting of blood, saliva, urine, tissue fluids, and exhaled breath. The blood sample may be whole blood or a blood fraction. The blood fraction may be, in turn, serum or plasma.

In the context of the present invention, the term "kit of parts (in short: kit)" is understood to be any combination of at least some of the components identified herein, which are combined, coexisting spatially, to a functional unit, and which can contain further components.

### Embodiments of the invention

The present invention will now be further described. In the following passages, different aspects of the invention are defined in more detail.

The present inventors surprisingly found that H₂¹⁸O depleted water can be used in a method for determining (total) energy expenditure. Due to the administration of H₂¹⁸O depleted water to a subject, the natural concentration of H₂¹⁸O in body water is slightly reduced. The H₂¹⁸O depleted body water further equilibrates via the water ingested during food intake and CO₂ exchange during respiration until it reaches the original natural H₂¹⁸O concentration again. From this, the elimination rate of ¹⁸O and, thus, the CO₂ turnover can be determined. The CO₂ turnover then allows to determine the (total) energy expenditure.

H₂¹⁸O depleted water is significantly cheaper to produce than H₂¹⁸O enriched water. It is a waste product of ¹⁸O isotope enrichment. In this way, a cost reduction of the method by an estimated factor of 10 can be achieved. This also makes the method interesting for private consumption (sport, training, nutrition, and/or lifestyle).

The method performed by the present inventors allows the determination of energy expenditure with high accuracy and precision. It can also be used to calibrate the inaccurate methods described in the prior art for each person individually.

In addition, the present inventors surprisingly found that ¹⁷O can be used as a tracer for background correction. Due to the additional use of H₂¹⁷O depleted or enriched water, the detection sensitivity can be improved.

Thus, in a first aspect, the present invention relates to a combination comprising, consisting of, or consisting essentially of ²H₂O enriched water and H₂¹⁸O depleted water.

In one embodiment, the ²H₂O enriched water comprises between 0.02 % and 100 % ²H₂O (specified as ²H fraction). Particularly, the ²H₂O enriched water comprises between 10 % and 100 % ²H₂O.

In one (alternative or additional) embodiment, the H₂¹⁸O depleted water comprises between 0 % and < 0.2 % H₂¹⁸O (specified as ¹⁸O fraction). Particularly, the H₂¹⁸O depleted water comprises between 0% and < 0.18%.

For example, the combination comprises ²H₂O enriched water and H₂¹⁸O depleted water, wherein the ²H₂O enriched water comprises between 0.02 % and 100 % ²H₂O and wherein the H₂¹⁸O depleted water comprises between 0 % and < 0.2 % H₂¹⁸O.

In one preferred embodiment, the combination further comprises H₂¹⁷O enriched water and H₂¹⁷O depleted water.

Such a combination comprises ²H₂O and H₂¹⁷O enriched water and H₂¹⁸O and H₂¹⁷O depleted water. For example, the combination comprises ²H₂O and H₂¹⁷O enriched water and H₂¹⁸O and H₂¹⁷O depleted water, wherein the ²H₂O enriched water comprises between 0.02 % and 100 % ²H₂O and wherein the H₂¹⁸O depleted water comprises between 0 % and < 0.2 % H₂¹⁸O.

In one more preferred embodiment,
the ²H₂O enriched water is comprised/provided in a (first) composition and the H₂¹⁸O depleted water is comprised/provided in a (second/different) composition, or
the ²H₂O enriched water and the H₂¹⁸O depleted water are comprised/provided in a (single) composition.

Particularly, the present invention relates to a combination comprising
(i) a (first) composition comprising ²H₂O enriched water and a (second/different) composition comprising H₂¹⁸O depleted water, or
(ii) a (single) composition comprising (a mixture of) ²H₂O enriched water and H₂¹⁸O depleted water.

Thus, the combination may consist of/represent a (first) composition comprising ²H₂O enriched water and a (second/different) composition comprising H₂¹⁸O depleted water or a (single) composition comprising (a mixture of) ²H₂O enriched water and H₂¹⁸O depleted water.

More particularly, the present invention relates to a combination comprising
(i) a (first) composition comprising ²H₂O enriched water and a (second/different) composition comprising H₂¹⁸O depleted water, or
(ii) a (single) composition comprising (a mixture of) ²H₂O enriched water and H₂¹⁸O depleted water,
wherein the ²H₂O enriched water comprises between 0.02 % and 100 % ²H₂O and wherein the H₂¹⁸O depleted water comprises between 0 % and < 0.2 % H₂¹⁸O.

In one even more preferred embodiment, the (first/second/single) composition (further) comprises H₂¹⁷O enriched water and H₂¹⁷O depleted water.

Particularly, the combination comprises
(i) a (first) composition comprising ²H₂O and H₂¹⁷O enriched water and a (second/different) composition comprising H₂¹⁸O and H₂¹⁷O depleted water, or
(ii) a (single) composition comprising (a mixture of) ²H₂O and H₂¹⁷O enriched water and H₂¹⁸O and H₂¹⁷O depleted water.

In this respect, it should be noted that in the (single) composition comprising (a mixture of) ²H₂O enriched water and H₂¹⁸O depleted water, the H₂¹⁷O content (nearly) approaches the natural ¹⁷O content.

More particularly, the combination comprises
(i) a (first) composition comprising ²H₂O and H₂¹⁷O enriched water and a (second/different) composition comprising H₂¹⁸O and H₂¹⁷O depleted water, or
(ii) a (single) composition comprising (a mixture of) ²H₂O and H₂¹⁷O enriched water and H₂¹⁸O and H₂¹⁷O depleted water,
wherein the ²H₂O enriched water comprises between 0.02 % and 100 % ²H₂O and wherein the H₂¹⁸O depleted water comprises between 0 % and < 0.2 % H₂¹⁸O.

In this respect, it should be noted that in the (single) composition comprising (a mixture of) ²H₂O enriched water and H₂¹⁸O depleted water, the H₂¹⁷O content (nearly) approaches the natural ¹⁷O content.

The combination, more specifically the first composition, the second composition, and/or the single composition, of the first aspect, is (are) are preferably sterilized. The sterilization may be performed by distillation or filtration through a filtering system, e.g. through a 0.22-µm filtering system.

Further, the combination, more specifically the first composition, the second composition, and/or the single composition, of the first aspect, is (are) are preferably provided as liquid/in liquid form, e.g. as solution(s) such as drinking solution(s).

Furthermore, the combination, more specifically the first composition, the second composition, and/or the single composition, of the first aspect, is (are) preferably administered to a subject for measuring/determining (total) energy expenditure of the subject. Particularly, the subject is a human being or an animal. More particularly, the subject is a human being. The administration may take place by oral ingestion or injection. Preferably, the administration takes place by oral ingestion.

In addition, the combination, more specifically the first composition, the second composition, and/or the single composition, of the first aspect, is (are) preferably comprised in an administration container. This administration container should be easy to drink from. Said administration container should be washed with water after the dose is consumed and the washing water should also be consumed. This ensures that the entire dose is received (most containers will not deliver the total dose without washing). For children and the elderly, a straw can be used in order to prevent water loss during administration.

In a second aspect, the present invention relates to a kit for (*in vitro*) measuring (total) energy expenditure of a subject, said kit comprises:
(i) the combination of the first aspect.

Preferably, the kit further comprises additional means for measuring (total) energy expenditure of a subject.

More preferably, the additional means encompass:
(ii) one or more sample collection tubes, particularly with machine-readable codes for each sample collection time point,
(iii) one or more (administration) containers containing the combination of the first aspect, e.g. one container for the first composition and one container for the second composition, or one container for the single composition of the first aspect,
(iv) a smartphone application to remind for sample collection and/or to document collection time and/or to transfer this data to a central lab or to collect data from the central lab analysis, and/or
(v) packaging material.

Even more preferably, the kit further comprises
(vi) a label or packaging insert contained within the packaging material indicating that the kit is for (*in vitro*) measuring energy expenditure of a subject.

The administration container(s) is (are) preferably sterilized. The administration container(s) should be easy to drink from.

The label or packaging insert may further comprise information/instructions for sample collection and/or for taking the combination, more specifically the first composition, second composition and/or single composition, of the first aspect. For example, the label or packaging insert may comprise the information that the (administration) container(s) should be emptied completely and washed with water after the dose is consumed by the subject. The washing water should also be consumed. This ensures that the entire dose is received (most containers will not deliver the total dose without washing). Particularly, the subject is a human being or an animal. More particularly, the subject is a human being.

In a third aspect, the present invention relates to the (*in vitro*) use of the combination of the first aspect or the kit of the second aspect for measuring energy expenditure of a subject. In particular, the energy expenditure is the total energy expenditure (TEE). Preferably, the subject is a human being or an animal. More preferably, the subject is a human being.

The present inventors surprisingly found that H₂¹⁸O depleted water can be used in a method for determining (total) energy expenditure. Due to the administration of H₂¹⁸O depleted water to a subject, the natural concentration of H₂¹⁸O in body water is slightly reduced. The H₂¹⁸O depleted body water further equilibrates via the water ingested during food intake and CO₂ exchange during respiration until it reaches the original natural H₂¹⁸O concentration again. From this, the elimination rate of ¹⁸O and, thus, the CO₂ turnover can be determined. The CO₂ turnover then allows to determine the (total) energy expenditure.

H₂¹⁸O depleted water is significantly cheaper to produce than H₂¹⁸O enriched water. It is a waste product of ¹⁸O isotope enrichment. In this way, a cost reduction of the method by an estimated factor of 10 can be achieved. This also makes the method interesting for private consumption (sport, training, nutrition, and/or lifestyle).

The method performed by the present inventors allows the determination of energy expenditure which high accuracy and precision. It can also be used to calibrate the inaccurate methods described in the prior art for each person individually.

Thus, in a fourth aspect, the present invention relates to a (an *in vitro*) method of measuring (total) energy expenditure of a subject comprising the steps of:
(i) measuring the ²H/¹H ratio and the ¹⁸O/¹⁶O ratio in a body water sample obtained from a subject at a first point in time (pre-dose baseline ratios),
(ii) measuring the ²H/¹H ratio and the ¹⁸O/¹⁶O ratio in a body water sample obtained from the subject at a second point in time (post-dose ratios) and in at least one further body water sample obtained from said subject at a later point in time, wherein the subject is a subject to whom a combination comprising, consisting essentially of, or consisting of ²H₂O enriched water and H₂¹⁸O depleted water had been administered,
(iii) determining a combined value of flux of body water and exhaled/released carbon dioxide (CO₂) from a change in measured ¹⁸O/¹⁶O ratios over time and a value of flux of body water alone from a change in measured ²H/¹H ratios over time, and
(iv) calculating the (total) energy expenditure on the basis of the exhaled/released CO₂ over time calculated from the combined values determined in step (iii).

There are two approaches for carrying out the above method: the two-point approach and the multi-point (more than two-point) approach. The two-point approach in its minimal form requires three body water samples, a pre-dose baseline body water sample (before the administration of the combination comprising ²H₂O enriched water and H₂¹⁸O depleted water), a post-dose body water sample (preferably at the day of dosing after the isotopes have equilibrated throughout the body of the subject) and a final body water sample taken at the end of the analysis (that is, for example, between day 10 to 14). The multi-point (more than two-point) approach in its most extreme form generally involves taking a pre-dose baseline body water sample (before the administration of the combination comprising ²H₂O enriched water and H₂¹⁸O depleted water) and body water samples every day after intake of the dose until the end of the sampling period.

An advantage of the two-point approach is that it requires fewer samples and provides the more exact estimate of (total) energy expenditure. The disadvantage of this approach is that it is prone to analytical or sampling disadvantages of the protocol and provides little data for assessing variance and internal consistency. In contrast, the multi-point approach has the advantage of data averaging and, thus, minimizes analytical errors. The disadvantage of this approach is that it requires more samples and, thus, places a greater burden on the subject tested.

In this respect, it should be noted that the pre-dose ratio is measured before the administration of the combination comprising ²H₂O enriched water and H₂¹⁸O depleted water, the post-dose ratio is measured after ²H₂O and H₂¹⁸O has equilibrated throughout the body of the subject, and the final ratio is measured at the end of the study.

In one embodiment, the ²H₂O enriched water comprises between 0.02 % and 100 % ²H₂O (specified as ²H fraction). Particularly, the ²H₂O enriched water comprises between 10 % and 100 % ²H₂O.

In one (alternative or additional) embodiment, the H₂¹⁸O depleted water comprises between 0 % and < 0.2 % H₂¹⁸O (specified as ¹⁸O fraction). Particularly, the H₂¹⁸O depleted water comprises between 0 % and < 0.18 % H₂¹⁸O.

In one example, the combination comprises ²H₂O enriched water and H₂¹⁸O depleted water, wherein the ²H₂O enriched water comprises between 0.02 % and 100 % ²H₂O and wherein the H₂¹⁸O depleted water comprises between 0 % and < 0.2 % H₂¹⁸O.

In one preferred embodiment, the combination comprising ²H₂O enriched water and H₂¹⁸O depleted water had been administered to the subject after the first point in time. More specifically, the combination comprising ²H₂O enriched water and H₂¹⁸O depleted water had been administered to the subject after the first point in time and before the second point in time. The first point in time may be immediately, e.g. 1, 2, 3, 4, 5, 6, 7, 8, 9, or 10 minutes or within 1 or 2 hours, before the administration of a combination comprising ²H₂O enriched water and H₂¹⁸O depleted water to the subject.

Further, the second point in time may be between 1 and 5 hours, such as between 2 to 4 hours, e.g. 1, 2, 3, 4, or 5 hours, after the administration of a combination comprising ²H₂O enriched water and H₂¹⁸O depleted water to the subject. After this time period, it is assumed that the isotopes have equilibrated throughout the body of the subject.

Furthermore, the time period between the second point in time and the later point in time may be between 1 and 14 days (2 weeks), e.g. 1, 2, 3, 4, 5, 6, 7, 8, 9, 10, 11, 12, 13, or 14 days (2 weeks).

In one more preferred embodiment,
the ²H₂O enriched water is comprised in a composition and the H₂¹⁸O depleted water is comprised/provided in a (another/different) composition, or
the ²H₂O enriched water and the H₂¹⁸O depleted water are comprised/provided in a (single) composition.

Particularly, the combination comprises
(i) a composition comprising ²H₂O enriched water and a (another/different) composition comprising H₂¹⁸O depleted water, or
(ii) a (single) composition comprising (a mixture of) ²H₂O enriched water and H₂¹⁸O depleted water.

Thus, the combination may consist of/represent a composition comprising ²H₂O enriched water and a (another/different) composition comprising H₂¹⁸O depleted water or a (single) composition comprising (a mixture of) ²H₂O enriched water and H₂¹⁸O depleted water.

More particularly,
(i) the composition comprising ²H₂O enriched water had been administered first and the (other/different) composition comprising H₂¹⁸O depleted water had been administered afterwards,
(ii) the composition comprising H₂¹⁸O depleted water had been administered first and the (other/different) composition comprising ²H₂O enriched water had been administered afterwards, or
(iii) the composition comprising ²H₂O enriched water and the (other/different) composition comprising H₂¹⁸O depleted water had been administered at the same time.

Thus, the compositions can be administered concurrently or consecutively.

The isotope ¹⁷O is preferably used herein as a new, additional tracer for the determination of the (total) energy expenditure. In contrast to the investigation of the absolute oxygen isotope ratios, which are subject to mass-dependent dilution and fractionation processes in the body, the Δ¹⁷O signal behaves conservatively and can, therefore, be used as an additional, source-specific tracer to correct disturbing influences in the determination of (total) energy consumption. The ¹⁷O excess can be determined from the parallel measurement of all three oxygen isotopes (¹⁶O, ¹⁷O, and ¹⁸O). In addition, the ¹⁷O/¹⁶O ratio can be determined.

Thus, in one even more preferred embodiment, the subject is a subject to whom a combination further comprising H₂¹⁷O enriched water and H₂¹⁷O depleted water had been administered.

Such a combination comprises, consists essentially of, or consists of ²H₂O and H₂¹⁷O enriched water and H₂¹⁸O and H₂¹⁷O depleted water. For example, the combination comprises ²H₂O and H₂¹⁷O enriched water and H₂¹⁸O and H₂¹⁷O depleted water, wherein the ²H₂O enriched water comprises between 0.02 % and 100 % ²H₂O and wherein the H₂¹⁸O depleted water comprises between 0 % and < 0.2 % H₂¹⁸O.

Particularly, the ¹⁷O/¹⁶O ratio and/or the ¹⁷O-excess is further measured (in step (i)) in a body water sample obtained from a subject at a first point in time (baseline ratio).

More particularly, the ¹⁷O/¹⁶O ratio and/or the ¹⁷O-excess is further measured (in step (ii)) in a body water sample obtained from the subject at a second point in time (plateau ratio) and in at least one further body water sample obtained from said subject at a later point in time.

Even more particularly, a reference value of isotopic background fluctuation is further determined (in step (iii)) from a change in measured ¹⁷O/¹⁶O ratios and/or the ¹⁷O-excess over time.

Still even more particularly, the reference value of isotopic background fluctuation is used to adjust the change in measured ¹⁸O/¹⁶O ratio and/or ²H/¹H ratio over time. In this way, natural fluctuations of the isotopes ¹⁸O and ²H, which may influence the measuring accuracy, can be compensated.

In one still even more preferred embodiment, the combination comprising ²H₂O and H₂¹⁷O enriched water and H₂¹⁸O and H₂¹⁷O depleted water had been administered to the subject after the first point in time. More specifically, the combination comprising ²H₂O and H₂¹⁷O enriched water and H₂¹⁸O and H₂¹⁷O depleted water had been administered to the subject after the first point in time and before the second point in time.

The first point in time may be immediately, e.g. 1, 2, 3, 4, 5, 6, 7, 8, 9, or 10 minutes or within 1 or 2 hours, before the administration of a combination comprising ²H₂O and H₂¹⁷O enriched water and H₂¹⁸O and H₂¹⁷O depleted water to the subject.

Further, the second point in time may be between 1 and 5 hours, such as between 2 to 4 hours, e.g. 1, 2, 3, 4, or 5 hours, after the administration of a combination comprising ²H₂O and H₂¹⁷O enriched water and H₂¹⁸O and H₂¹⁷O depleted water to the subject. After this time period, the isotopes have equilibrated throughout the body of the subject.

Furthermore, the time period between the second point in time and the later point in time may be between 1 and 14 days (2 weeks), e.g. 1, 2, 3, 4, 5, 6, 7, 8, 9, 10, 11, 12, 13, or 14 days (2 weeks).

In one most preferred embodiment,
the ²H₂O and H₂¹⁷O enriched water is comprised/provided in a composition and the H₂¹⁸O and H₂¹⁷O depleted water is comprised in a (another/different) composition, or
the ²H₂O and H₂¹⁷O enriched water and the H₂¹⁸O and H₂¹⁷O depleted water are comprised/provided in a (single) composition.

Particularly, the combination comprises
(i) a composition comprising ²H₂O and H₂¹⁷O enriched water and a (different) composition comprising H₂¹⁸O and H₂¹⁷O depleted water, or
(ii) a (single) composition comprising (a mixture of) ²H₂O and H₂¹⁷O enriched water and H₂¹⁸O and H₂¹⁷O depleted water.

In this respect, it should be noted that in the (single) composition comprising (a mixture of) ²H₂O enriched water and H₂¹⁸O depleted water, the H₂¹⁷O content (nearly) approaches the natural ¹⁷O content.

More particularly,
(i) the composition comprising ²H₂O and H₂¹⁷O enriched water had been administered first and the composition comprising H₂¹⁸O and H₂¹⁷O depleted water had been administered afterwards,
(ii) the composition comprising H₂¹⁸O and H₂¹⁷O depleted water had been administered first and the composition comprising ²H₂O and H₂¹⁷O enriched water had been administered afterwards, or
(iii) the composition comprising ²H₂O and H₂¹⁷O enriched water and the composition comprising H₂¹⁸O and H₂¹⁷O depleted water had been administered at the same time.

In this respect, it should be noted that in the (single) composition comprising (a mixture of) ²H₂O enriched water and H₂¹⁸O depleted water, the H₂¹⁷O content (nearly) approaches the natural ¹⁷O content.

In one preferred example of the method of the fourth aspect, a base line sample of urine is analysed within one hour before administration of the ²H-enriched water and ¹⁸O-depleted water. After 2 to 4 hours, a second urine sample is measured (plateau sample). On days 1, 2, 4, 7, 8 and 9 further urine samples are analysed (or at least on days 1 and 9).

The skilled person is aware of techniques which allow the calculation of the (total) energy expenditure on the basis of the exhaled/released CO₂ over time calculated from the combined values determined in step (iii). In this respect, it is referred to IAEA Protocoll "Assessment of Body Composition and Total Energy Expenditure in Humans Using Stable Isotope Techniques" (© IAEA, 2009, ISBN 978-92-0-111708-3).

Further, the combination/composition(s) administered to the subject was (were) preferably sterilized, e.g. by distillation or filtration through a filtering system such as a 0.22-µm filtering system.

Furthermore, the combination/composition(s) administered to the subject was (were) preferably provided as liquid/in liquid form, e.g. as solution(s) such as drinking solution(s). Preferably, the administration had been taken place by oral ingestion or injection. More preferably, the administration had been taken place by oral ingestion.

In addition, the combination/composition(s) administered to the subject was (were) preferably comprised in an administration container.

The subject tested in the method of the fourth aspect may be any individual whose (total) energy expenditure is to be determined/measured. In particular, the subject is a hobby or professional athlete, an obese person, or a person suffering from a metabolic disorder such as diabetes. Preferably, the subject tested in the fourth aspect of the present invention is a human being or an animal. The animal may be selected from the group consisting of a dog, cat, sheep, goat, cow, horse, and pig. More preferably, the subject is a human being.

The ratios in the method of the fourth aspect are preferably determined using optical spectroscopy, laser spectroscopy, or mass spectroscopy such as isotope ratio mass spectroscopy. In particular, the optical spectroscopy comprises cavity ring-down spectroscopy (CRDS) and off-axis integrated cavity output spectroscopy (OA-ICOS).

The body water sample analysed in the method of the fourth aspect refers to any watery/aqueous sample taken from a subject, e.g. human being. In other words, the body water (also designated as body fluid or bodily fluid) is any liquid within the body of a subject, e.g. human being. A body water sample can be obtained, for example, by collection of any of urine, blood, saliva and exhaled breath from a subject. Preferably, the body water sample is selected from the group consisting of blood, saliva, urine, tissue fluids, and exhaled breath. The blood sample may be whole blood or a blood fraction. The blood fraction may be, in turn, serum or plasma. More preferably, the body water sample is urine.

It is specifically preferred that the combination of the first aspect had been administered to the subject tested in the method of the fourth aspect.

Dietary intake of athletes (in particular of professional athletes) must match training volume to fuel physiological demands. Inadequate energy is detrimental to bone health, immune health, cognitive performance, as well as exercise performance in physically active populations. In particular, adequate dietary intake is important for promoting adaptation and prevention of musculoskeletal injury in response to large volumes of physical training.

Not only power or endurance training is important. It is often important to include strength, power, endurance, and functional training.

Usually, athletes (in particular professional athletes) have unique fueling needs due to the large variety in training intensity and duration. Strength and power athletes have, for example, higher protein needs than endurance athletes and endurance athletes have, for example, higher carbohydrate and fat intake needs than strength and power athletes. Clearly understanding training volume and nutrition intakes of athletes (in particular professional athletes) is, thus, critical in ensuring training success.

Accordingly, in a fifth aspect, the present invention relates to a method of training optimization comprising the steps of:
(i) carrying out the method of the fourth aspect, thereby measuring the (total) energy expenditure of a subject, and
(ii) adapting the training of the subject on the basis of the (total) energy expenditure measured in step (i).

The world is currently experiencing a nutrition transition in which energy undernutrition coexists with energy overnutrition as a consequence of significant lifestyle changes. Both industrialized and developing societies are suffering the burden of obesity related to inactivity and excess of energy consumption and co-morbid conditions including hyperlipidaemias, insulin resistance, type 2 diabetes, cardiovascular diseases and cancer. There is also increasing evidence of the link between undernutrition in utero and obesity and chronic disease in later life. In either under- or overnutrition, there is an increasing need for nutritional status methodologies that provide reliable results for risk assessment and for the evaluation of nutrition and physical activity intervention programmers. Such measures require assessment of (total) energy expenditure.

Thus, in a sixth aspect, the present invention relates to a method of diet optimization comprising the steps of:
(i) carrying out the method of the fourth aspect, thereby measuring the (total) energy expenditure of a subject, and
(ii) adapting the diet of the subject on the basis of the (total) energy expenditure measured in step (i).

Thus, in a seventh aspect, the present invention relates to a method of obesity therapy optimization comprising the steps of:
(i) carrying out the method of the fourth aspect, thereby measuring the (total) energy expenditure of a subject, and
(ii) adapting the obesity therapy of the subject on the basis of the (total) energy expenditure measured in step (i).

The present invention is further illustrated by the following examples and Figures, however, without being restricted thereto.

### BRIEF DESCRIPTION OF THE FIGURES

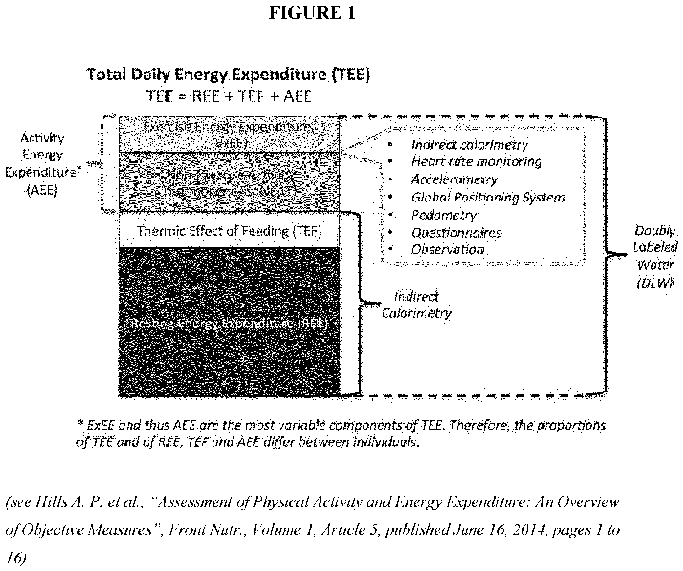

**Figure 1****:** Shows the components of total energy expenditure and measurement approaches (see Hills A. P. et al., "Assessment of Physical Activity and Energy Expenditure: An Overview of Objective Measures", Front Nutr., Volume 1, Article 5, published June 16, 2014, pages 1 to 16).
**Figure 2****:** Shows the Doubly Labeled Water method which calculates the energy consumption/expenditure via the carbon dioxide release. For this purpose, a certain amount of water, labelled hydrogen (²H) and oxygen (¹⁸O) isotopes is added orally. While the hydrogen (²H) isotope leaves the body via various fluids (sweat, urine, respiration), the oxygen isotope (¹⁸O) bind to carbon ions in addition to hydrogen and leave the body via carbon dioxide (CO₂). This results in a much higher elimination rate for the oxygen (¹⁸O) than for the hydrogen isotope (²H). The difference between the eliminated and remaining isotopes allows the calculation of the carbon dioxide release (CO₂) and the associated (total) energy consumption/expenditure within a certain period of time (taken from "Emerging Technologies for Nutrition Research: Potential for Assessing Military Performance Capability", paragraph 12 "Doubly labeled water for energy expenditure", (1997)).

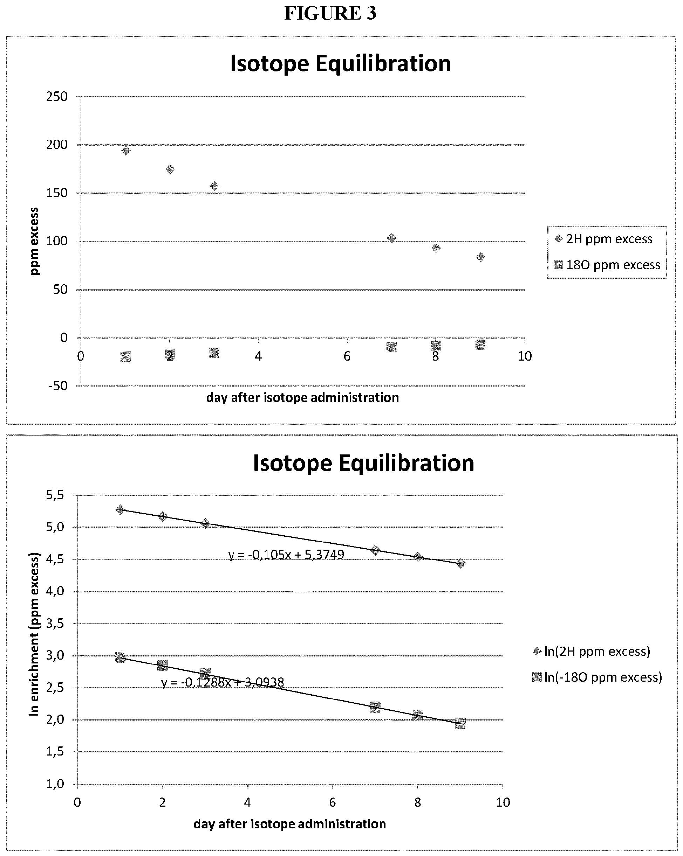

**Figure 3****:** Shows simulated data for the isotope equilibration using an ²H-enriched water and a ¹⁸O depleted water for the doubly labelled water method. Experimental design is described in experiment 1. Both isotopes are approaching zero ppm excess over time. ²H enrichment decreases and ¹⁸O enrichment increases to finally equilibrate with the natural abundance of the isotopes. The isotope elimination rates according to the IAEA protocol (ISBN 978-92-0-111708-3) can be calculated from the slope of the natural logarithm of the ppm excess values vs. time after isotope administration. For ¹⁸O the natural logarithm of the negative ppm excess value has to be used.

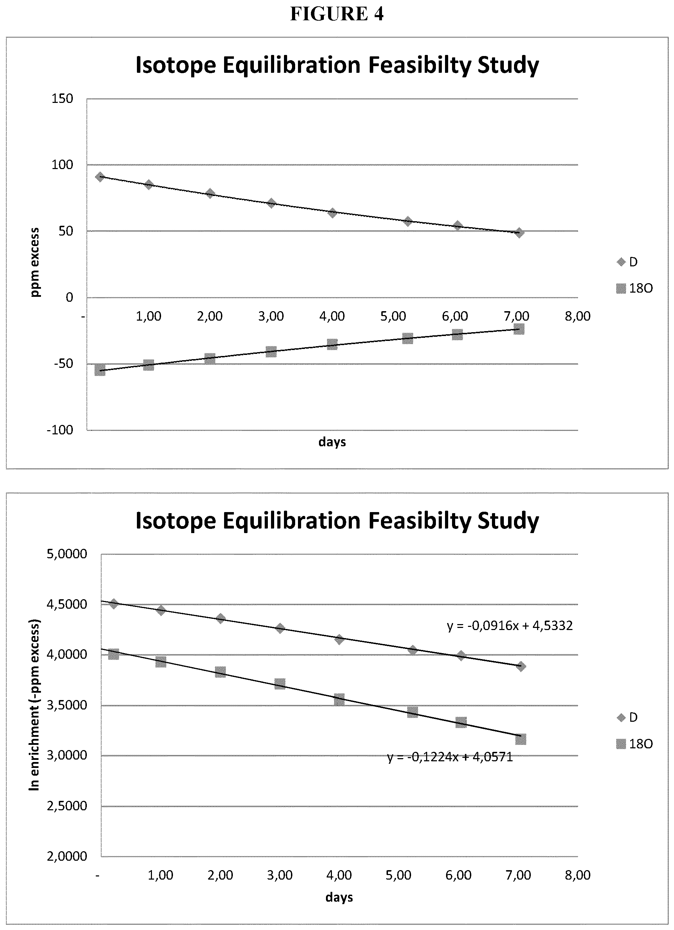

**Figure 4****:** Shows data from a feasibility study described in the examples. The results confirm that the exact course of ¹⁸O enrichment after administration of ¹⁸O-depleted water can be measured by laser spectrometry within at least 7 days after administration. This is prerequisite for the determination of the total energy expenditure.

### EXAMPLES

### 1. Example

Testing protocol for the determination of total energy expenditure (TTE) by the DLW method using ²H-enriched water and ¹⁸O-depleted water:
A base line sample of urine is analysed within one hour before administration of the ²H-enriched water and ¹⁸O-depleted water (body weight of the test person 70kg). According to the IAEA protocol, the test person gets 0.12g/kg body weight of 99 at.% ²H enriched water (=8.4 g). Additionally, the test person gets a dose of 800 ml of to 0.1 at.% ¹⁸O-depleted water . The ²H-enriched water and the ¹⁸O-depleted water has to be drunken within 20 min. After 2-4 hours, a second urine sample is measured (plateau sample). On day 1, 2, 4, 7, 8 and 9 further urine samples are analysed (or at least on day one and on day 9). ¹⁸O/¹⁶O and ²H/¹H ratios can optionally be adjusted by ¹⁷O excess analysis. The isotope elimination rates (k_{O} and k_{D}) can be calculated from the slope of the natural logarithm of the ppm excess values vs. time after isotope administration. Isotope dilution space at time of dosing is calculated from the intercept of the ²H-plot. For ¹⁸O, the natural logarithm of the negative ppm excess value has to be used (see **Figure 3****,** simulated data). For the simulated data, an isotope dilution space of 1944,4 mol, k_{D} of -0.1050 d⁻¹ and k_{O} of -0.1288d⁻¹ can be calculated. CO₂ production rate and total energy expenditure can finally be calculated according to the IAEA protocol (*ISBN* 978-92-0-111708-3, chapter 6.3. "Calculation of TEE". For the simulated data, a CO₂ production of 404,5l/day and a TEE of 2300,7 kcal/day can be calculated (expected respiratory exchange ratio RQ= 0.85).

Sample preparation: Before analysis the samples have to be prepared for instrument analysis. Urine and plasma samples should be centrifuged and ultrafiltrated.

Isotope measurement: Isotope measurements are performed using a commercially available laser spectrometer (L2140-i Liquid Water Isotope Analyzer, Picarro Inc.). The instrument allows high-precision determination of hydrogen isotopes ratio (δ²H) as well as oxygen isotope ratios (δ¹⁸O and δ¹⁷O) in water and body fluid samples. Samples are injected from a liquid autosampler into a vaporizer module and the water vapor formed is transferred to the laser spectrometer. Dry nitrogen is used as carrier gas. Isotope measurements can be performed in either normal mode or ¹⁷ O-excess mode. In normal mode, only δ²H and δ¹⁸O are determined, while ¹⁷O-excess mode allows simultaneous measurement of δ²H, δ¹⁸O, δ¹⁷O, and ¹⁷ O-excess respectively. The normalization of the isotopic measurements to the VSMOW-SLAP scale are performed using internal working standards previously calibrated against the international reference materials VSMOW and SLAP. The ppm values are calculated form the normalized isotope delta values according to the IAEA protocol.

### 2. Example

Testing protocol for the determination of total energy expenditure (TTE) by the DLW method using ²H-enriched water and ¹⁸O-depleted water:
A base line sample of urine is analysed within one hour before administration of the ²H-enriched water and ¹⁸O-depleted water (body weight of the test person 70kg). According to the IAEA protocol, the test person gets 0.12g/kg body water of 99 at.% ²H enriched water (=4.2 g). Additionally, the test person gets a dose of 2000 ml of to 0.1 at.% ¹⁸O-depleted water . The ²H-enriched water and the ¹⁸O-depleted water has to be drunken within 60 min. After 5 hours, a second urine sample is measured (plateau sample). On day 1, 2, 4, 7, 8 and 9 further urine samples are analysed (or at least on day one and on day 9). ¹⁸O/¹⁶O and ²H/¹H ratios can optionally be adjusted by ¹⁷O excess analysis. The isotope elimination rates (kO and kD) can be calculated from the slope of the natural logarithm of the ppm excess values vs. time after isotope administration. Isotope dilution space at time of dosing is calculated from the intercept of the ²H-plot. For ¹⁸O, the natural logarithm of the negative ppm excess value has to be used (see **Figure 3****,** simulated data). For the simulated data, an isotope dilution space of 1944,4 mol, kD of -0.1050 d-1 and kO of -0. 1288d-1 can be calculated. CO₂ production rate and total energy expenditure can finally be calculated according to the IAEA protocol (ISBN 978-92-0-111708-3, chapter 6.3. "Calculation of TEE". For the simulated data, a CO₂ production of 404,5l/day and a TEE of 2300,7 kcal/day can be calculated (expected respiratory exchange ratio RQ= 0.85).

Isotope measurement: Isotope measurements are performed using a commercially available laser spectrometer (L2140-i Liquid Water Isotope Analyzer, Picarro Inc.). The instrument allows high-precision determination of hydrogen isotopes ratio (δ²H) as well as oxygen isotope ratios (δ¹⁸O and δ¹⁷O) in water and body fluid samples. Samples are injected from a liquid autosampler into a vaporizer module and the water vapor formed is transferred to the laser spectrometer. Dry nitrogen is used as carrier gas. Isotope measurements can be performed in either normal mode or ¹⁷O-excess mode. In normal mode, only δ²H and δ¹⁸O are determined, while ¹⁷O-excess mode allows simultaneous measurement of δ²H, δ¹⁸O, δ¹⁷O, and ¹⁷ O-excess respectively. The normalization of the isotopic measurements to the VSMOW-SLAP scale are performed using internal working standards previously calibrated against the international reference materials VSMOW and SLAP. The ppm values are calculated form the normalized isotope delta values according to the IAEA protocol.

### 3. Example

Feasibility study:
A base line sample of urine was analyzed within one hour before administration of the ²H-enriched water and ¹⁸O-depleted water (body weight of the test person 70kg). According to the IAEA protocol, the test person got 0.12g/kg body water of 99 at.% ²H enriched water (=4.3 g). Additionally, the test person got a dose of 2000 ml of to 0.074 at.% ¹⁸O-depleted water. The ²H-enriched water and the ¹⁸O-depleted water were drunken within 60 min. After 5 hours, a second urine sample was measured (plateau sample). On day(s) 1, 2, 3, 4, 5, 6 and 7, further urine samples were analyzed according to the described method by laser spectrometry. **Figure 4****,** Tables 1 and 2 show the results of these measurements. The results confirm that the exact course of ¹⁸O enrichment after administration of ¹⁸O-depleted water can be measured by laser spectrometry within at least 7 days after administration. This is prerequisite for the determination of the total energy expenditure. The total energy expenditure (TEE) can be calculated with these data. Calculation of TEE can be performed according to Example 2.

**Table 1: Test person, doses**

| | |
|---|---|
| Age | 48 years |
| Weight | 72,5 kg |
| Sex | Male |
| Height | 172 cm |
| ¹⁸O depleted water Dose | 2000 ml |
| ¹⁸O abundance Dose | 701.3 ppm |
| ¹⁸O abundance Basline | 1.993.6 ppm |
| ²H enriched water Dose (99% enrichment) | 4.2 g |
| ²H abundance Baseline | 149.9 ppm |

**Table 2: Results laser spectroscopy**

| | measured abundance ¹⁸O [ppm] | measured [ppm] xs ¹⁸O | measured abundance ²H [ppm] | measured [ppm] xs ²H |
|---|---|---|---|---|
| Baseline | 1.993,6 | | 149,9 | |
| 5h | 1.938,6 | -54,98 | 240,6 | 90,7 |
| day 1 | 1.942,6 | -50,97 | 234,9 | 85,0 |
| day 2 | 1.947,5 | -46,09 | 228,3 | 78,4 |
| day 3 | 1.952,7 | -40,85 | 221,0 | 71,1 |
| day 4 | 1.958,3 | -35,21 | 213,4 | 63,5 |
| day 5 | 1.962,7 | -30,89 | 207,1 | 57,2 |
| day 6 | 1.965,6 | -27,97 | 204,0 | 54,2 |
| day 7 | 1.969,9 | -23,70 | 198,6 | 48,7 |

## Claims

1. A combination comprising ²H₂O enriched water and H₂¹⁸O depleted water.

2. The combination of claim 1, wherein the ²H₂O enriched water comprises between 0.02 % and 100 % ²H₂O (specified as ²H fraction).

3. The combination of claims 1 or 2, wherein the H₂¹⁸O depleted water comprises between 0% and < 0.2 % H₂¹⁸O (specified as ¹⁸O fraction).

4. The combination of any one of claims 1 to 3, wherein the combination further comprises H₂¹⁷O enriched water and H₂¹⁷O depleted water.

5. The combination of any one of claims 1 to 3, wherein
the ²H₂O enriched water is comprised in a (first) composition and the H₂¹⁸O depleted water is comprised in a (second) composition, or
the ²H₂O enriched water and the H₂¹⁸O depleted water are comprised in a (single) composition.

6. The combination of claim 5, wherein the (first/second/single) composition (further) comprises H₂¹⁷O enriched water and H₂¹⁷O depleted water.

7. A kit for measuring energy expenditure of a subject, said kit comprises:
the combination of any one of claims 1 to 6.

8. Use of the combination of any one of claims 1 to 6 or the kit of claim 7 for measuring energy expenditure of a subject.

9. A method of measuring energy expenditure of a subject comprising the steps of:
(i) measuring the ²H/¹H ratio and the ¹⁸O/¹⁶O ratio in a body water sample obtained from a subject at a first point in time (pre-dose baseline ratios),
(ii) measuring the ²H/¹H ratio and the ¹⁸O/¹⁶O ratio in a body water sample obtained from the subject at a second point in time (post-dose ratios) and in at least one further body water sample obtained from said subject at a later point in time, wherein the subject is a subject to whom a combination comprising ²H₂O enriched water and H₂¹⁸O depleted water had been administered,
(iii) determining a combined value of flux of body water and exhaled/released carbon dioxide (CO₂) from a change in measured ¹⁸O/¹⁶O ratios over time and a value of flux of body water alone from a change in measured ²H/¹H ratios over time, and
(iv) calculating the energy expenditure on the basis of the exhaled/released CO₂ over time calculated from the combined values determined in step (iii).

10. The method of claim 9, wherein the ²H₂O enriched water comprises between 0.02 % and 100 % ²H₂O.

11. The method of claims 9 or 10, wherein the H₂¹⁸O depleted water comprises between 0 % and < 0.2 % H₂¹⁸O.

12. The method of any one of claims 9 to 11, wherein the subject is a subject to whom a combination further comprising H₂¹⁷O enriched water and H₂¹⁷O depleted water had been administered.

13. A method of training optimization comprising the steps of:
(i) carrying out the method of any one of claims 9 to 12, thereby measuring the energy expenditure of a subject, and
(ii) adapting the training of the subject on the basis of the energy expenditure measured in step (i).

14. A method of diet optimization comprising the steps of:
(i) carrying out the method of any one of claims 9 to 12, thereby measuring the energy expenditure of a subject, and
(ii) adapting the diet of the subject on the basis of the energy expenditure measured in step (i).

15. A method of obesity therapy optimization comprising the steps of:
(i) carrying out the method of any one of claims 9 to 12, thereby measuring the energy expenditure of a subject, and
(ii) adapting the obesity therapy of the subject on the basis of the energy expenditure measured in step (i).

## Patentansprüche

1. Kombination umfassend mit ²H₂O angereichertes Wasser und H₂¹⁸O abgereichertes Wasser.

2. Kombination nach Anspruch 1, wobei das mit ²H₂O angereicherte Wasser zwischen 0,02 % und 100 % ²H₂O (angegeben als ²H-Fraktion) umfasst.

3. Kombination nach Anspruch 1 oder 2, wobei das mit H₂¹⁸O abgereicherte Wasser zwischen 0 % und < 0,2 % H₂¹⁸O (angegeben als ¹⁸O-Fraktion) umfasst.

4. Kombination nach einem der Ansprüche 1 bis 3, wobei die Kombination ferner mit H₂¹⁷O angereichertes Wasser und H₂¹⁷O abgereichertes Wasser umfasst.

5. Kombination nach einem der Ansprüche 1 bis 3, wobei
das mit ²H₂O angereicherte Wasser in einer (ersten) Zusammensetzung enthalten ist und das H₂¹⁸O abgereicherte Wasser in einer (zweiten) Zusammensetzung enthalten ist, oder
das mit ²H₂O angereicherte Wasser und das H₂¹⁸O abgereicherte Wasser in einer (einzigen) Zusammensetzung enthalten sind.

6. Kombination nach Anspruch 5, wobei die (erste/zweite/einzige) Zusammensetzung (weiterhin) mit H₂¹⁷O angereichertes Wasser und H₂¹⁷O abgereichertes Wasser umfasst.

7. Kit zur Messung des Energieverbrauchs einer Person, wobei das Kit umfasst:
die Kombination nach einem der Ansprüche 1 bis 6.

8. Verwendung der Kombination nach einem der Ansprüche 1 bis 6 oder des Kits nach Anspruch 7 zur Messung des Energieverbrauchs einer Person.

9. Verfahren zur Messung des Energieverbrauchs einer Person, umfassend die folgenden Schritte:
(i) Messen des ²H/¹H Verhältnisses und des ¹⁸O/¹⁶O Verhältnisses in einer Körperwasserprobe, die einer Person zu einem ersten Zeitpunkt entnommen wurde (Ausgangsverhältnisse vor der Verabreichung),
(ii) Messen des ²H/¹H Verhältnisses und des ¹⁸O/¹⁶O Verhältnisses in einer Körperwasserprobe, die der Person zu einem zweiten Zeitpunkt entnommen wurde (Verhältnisse nach der Verabreichung), und in mindestens einer weiteren Körperwasserprobe, die der Person zu einem späteren Zeitpunkt entnommen wurde,
wobei die Person eine Person ist, der eine Kombination umfassend mit ²H₂O angereichertes Wasser und H₂¹⁸O abgereichertes Wasser verabreicht wurde,
(iii) Bestimmen eines kombinierten Wertes des Körperwasserflusses und des ausgeatmeten/freigesetzten Kohlendioxids (CO₂) aus einer Veränderung des gemessenen ¹⁸O/¹⁶O Verhältnisses über die Zeit und eines Wertes des Körperwasserflusses allein aus einer Veränderung des gemessenen ²H/¹H Verhältnisses über die Zeit, und
(iv) Berechnen des Energieverbrauchs auf der Grundlage des ausgeatmeten/freigesetzten CO₂ über die Zeit, berechnet aus den in Schritt (iii) ermittelten kombinierten Werten.

10. Verfahren nach Anspruch 9, wobei das mit ²H₂O angereicherte Wasser zwischen 0,02 % und 100 % ²H₂O umfasst.

11. Verfahren nach Anspruch 9 oder 10, wobei das mit H₂¹⁸O abgereicherte Wasser zwischen 0% und < 0,2 % H₂¹⁸O umfasst.

12. Verfahren nach einem der Ansprüche 9 bis 11, wobei die Person eine Person ist, der eine Kombination, die ferner mit H₂¹⁷O angereichertes Wasser und H₂¹⁷O abgereichertes Wasser umfasst, verabreicht worden ist.

13. Verfahren zur Optimierung des Trainings, umfassend die Schritte:
(i) Durchführen des Verfahrens nach einem der Ansprüche 9 bis 12, wodurch der Energieverbrauch einer Person gemessen wird, und
(ii) Anpassen des Trainings der Person auf der Grundlage des in Schritt (i) gemessenen Energieverbrauchs.

14. Verfahren zur Optimierung der Ernährung, umfassend die Schritte:
(i) Durchführen des Verfahrens nach einem der Ansprüche 9 bis 12, wodurch der Energieverbrauch einer Person gemessen wird, und
(ii) Anpassen der Ernährung der Person auf der Grundlage des in Schritt (i) gemessenen Energieverbrauchs.

15. Verfahren zur Optimierung der Adipositas-Therapie, umfassend die Schritte:
(i) Durchführen des Verfahrens nach einem der Ansprüche 9 bis 12, wodurch der Energieverbrauch einer Person gemessen wird, und
(ii) Anpassen der Adipositas-Therapie der Person auf der Grundlage des in Schritt (i) gemessenen Energieverbrauchs.

## Revendications

1. Combinaison comprenant de l'eau enrichie en ²H₂O et de l'eau appauvrie en H₂¹⁸O.

2. Combinaison de la revendication 1, dans laquelle l'eau enrichie en ²H₂O comprend entre 0,02 % et 100 % de ²H₂O (spécifié comme fraction ²H).

3. Combinaison de la revendication 1 ou 2, dans laquelle l'eau appauvrie en H₂¹⁸O comprend entre 0 % et < 0,2 % de H₂¹⁸O (spécifié comme fraction ¹⁸O).

4. Combinaison de l'une quelconque des revendications 1 à 3, ladite combinaison comprenant en outre de l'eau enrichie en H₂¹⁷O et de l'eau appauvrie en H₂¹⁷O.

5. Combinaison de l'une quelconque des revendications 1 à 3, dans laquelle
l'eau enrichie en ²H₂O est comprise dans une (première) composition et l'eau appauvrie en H₂¹⁸O est comprise dans une (seconde) composition, ou
l'eau enrichie en ²H₂O et l'eau appauvrie en H₂¹⁸O sont comprises dans une (seule) composition.

6. Combinaison de la revendication 5, dans laquelle la (première/seconde/seule) composition comprend (en outre) de l'eau enrichie en H₂¹⁷O et de l'eau appauvrie en H₂¹⁷O.

7. Kit permettant de mesurer la dépense énergétique d'un sujet, ledit kit comprenant :
la combinaison de l'une quelconque des revendications 1 à 6.

8. Utilisation de la combinaison de l'une quelconque des revendications 1 à 6 ou kit de la revendication 7 permettant de mesurer la dépense énergétique d'un sujet.

9. Procédé de mesure de la dépense énergétique d'un sujet comprenant les étapes de :
(i) mesure du rapport ²H/¹H et du rapport ¹⁸O/¹⁶O dans un échantillon d'eau corporelle obtenu d'un sujet à un premier moment donné (rapports de référence pré-dose),
(ii) mesure du rapport ²H/¹H et du rapport ¹⁸O/¹⁶O dans un échantillon d'eau corporelle obtenu d'un sujet à un second moment donné (rapports post-dose) et dans au moins un autre échantillon d'eau corporelle obtenu dudit sujet à un moment donné ultérieur,
ledit sujet étant un sujet auquel une combinaison comprenant de l'eau enrichie en ²H₂O et de l'eau appauvrie en H₂¹⁸O a été administrée,
(iii) détermination d'une valeur combinée du flux d'eau corporelle et de dioxyde de carbone expiré/libéré (CO₂) à partir d'un changement des rapports ¹⁸O/¹⁶O mesurés au cours du temps et d'une valeur du flux d'eau corporelle seule à partir d'un changement des rapports ²H/¹H mesurés au cours du temps, et
(iv) calcul de la dépense énergétique sur la base du CO₂ expiré/libéré au cours du temps calculée à partir des valeurs combinées déterminées à l'étape (iii).

10. Procédé de la revendication 9, dans lequel l'eau enrichie en ²H₂O comprend entre 0,02 % et 100 % de ²H₂O.

11. Procédé de la revendication 9 ou 10, dans lequel l'eau appauvrie en H₂¹⁸O comprend entre 0 % et < 0,2 % de H₂¹⁸O.

12. Procédé de l'une quelconque des revendications 9 à 11, dans lequel le sujet est un sujet auquel une combinaison comprenant en outre de l'eau enrichie en H₂¹⁷O et de l'eau appauvrie en H₂¹⁷O a été administrée.

13. Procédé d'optimisation de l'entraînement comprenant les étapes de :
(i) mise en œuvre du procédé de l'une quelconque des revendications 9 à 12, mesurant ainsi la dépense énergétique d'un sujet, et
(ii) adaptation de l'entraînement du sujet sur la base de la dépense énergétique mesurée à l'étape (i).

14. Procédé d'optimisation de régime alimentaire comprenant les étapes de :
(i) mise en œuvre du procédé de l'une quelconque des revendications 9 à 12, mesurant ainsi la dépense énergétique d'un sujet, et
(ii) adaptation du régime alimentaire du sujet sur la base de la dépense énergétique mesurée à l'étape (i).

15. Procédé d'optimisation du traitement de l'obésité comprenant les étapes de :
(i) mise en œuvre du procédé de l'une quelconque des revendications 9 à 12, mesurant ainsi la dépense énergétique d'un sujet, et
(ii) adaptation du traitement de l'obésité du sujet sur la base de la dépense énergétique mesurée à l'étape (i).
